# EUROPEAN PATENT SPECIFICATION

(11) **EP 0 476 849 B1**
(45) Date of publication and mention of the grant of the patent: **05.03.1997**
(21) Application number: 91307746.7
(22) Date of filing: 22.08.1991
(51) Int. Cl.: C12N 9/18, C12Q 1/44

(54) **Human cytosolic phospholipase A2**
Menschliche Zytosolische Phospholipase A2
Phospholipase A2 cytosolique humaine

(30) Priority: 24.08.1990 US 573513
(43) Date of publication of application: 25.03.1992
(73) Proprietor: ELI LILLY AND COMPANY, Indianapolis, Indiana 46285 (US)
(72) Inventor: Kramer, Ruth Maria, Indianapolis, Indiana 46208 (US)
(74) Representative: Hudson, Christopher Mark

(56) References cited:
- WO-A-89/09818
- BIOCHEMICAL AND BIOPHYSICAL RESEARCH COMMUNICATIONS, vol. 170, no. 2, 31 July 1990, DULUTH MN, USA, pages 484-490; Y. YOSHIHARA ET AL.: 'Translocation of phospholipase A2 from cytosol to membranes in rat brain induced by calcium ions.'
- THE JOURNAL OF BIOLOGICAL CHEMISTRY, vol. 266, no. 8, 15 March 1991, BALTIMORE, USA, pages 5268-5272; R. KRAMER ET AL
- CELL, vol. 65, 14 June 1991, CAMBRIDGE MA, USA, pages 1043-1051; J. CLARK ET AL.: 'A novel arachidonic acid-selective cytosolic PLA2 contains a Ca2+ dependent translocation domain with homology to PKC and GAP.'

## Description

The present invention relates to a process for the isolation of cytosolic phospholipase A₂ enzymes of approximately 100,000 daltons. Specifically, this invention includes a novel human intracellular phospholipase A₂ enzyme isolated in substantially pure form from a human monoblast derived cell line.

Phospholipases have been known and studied for over 100 years and form a large class of ubiquitous enzymes that hydrolyze phosphoglycerides. Phospholipases are categorized into five groups (A₁, A₂, B, C and D) depending on the substrate bond that is cleaved.

Phospholipase A₂ (PLA₂) is the common name for phosphatide 2-acylhydrolase which catalyzes the hydrolysis of the sn-2-acyl ester bond of phosphoglycerides producing equimolar amounts of lysophospholipids and free fatty acids (Dennis, E. A., The Enzymes, Vol. 16, Academic Press, New York, (1983)). PLA₂ enzymes are found in all living species and form a diverse family of enzymes. Biochem. Biophys. Res. Comm. (1990) 170:484 discloses the purification of cytosolic phospholipase A2 from rat brain. Of the PLA₂s studied to date, the vast majority have a molecular weight of approximately 14 Kd, and their amino acid sequences show great homolgy.

The most abundant and commonly studied PLA₂s are the secreted forms. These enzymes are produced within the cell, packaged into secretory vesicles and later released into the extracellular environment where they aid in the digestion of biological material. In contrast, cytosolic PLA₂s are found in vanishingly small amounts, remain within the cell and serve in an entirely different capacity than the secreted form. Thorough investigation of cytosolic PLA₂s has been hampered by the extremely low concentration of these enzymes in cells (Vadas and Pruzanski, Lab. Investigation, 55, 4: 391 (1986)).

Cytosolic PLA₂s play a key regulatory role in the biosynthetic pathway leading to the formation of potent pro-inflammatory mediators known as platelet activating factors (PAFs) and eicosanoids. Eicosanoids include prostaglandins, thromboxanes, hydroxyeicosatetraenoic acid, leukotrienes and lipoxins (Mobilio and Marshall, Ann. Reports in Med. Chem., 24: 157 (1989)). Free arachidonic acid is the rate limiting precursor for the production of eicosanoids and is liberated from its membrane phospholipid stores by the action of particular cytosolic PLA₂s (Dennis, Drug Dev. Res., 10: 205, (1987)). The same enzymatic step also produces lysophospholipids which may be converted to form PAFs. At basal levels, cytosolic PLA₂s partake in the deacylation-reacylation reactions necessary to maintain the cellular phospholipid metabolism while receptor-mediated, increased PLA₂ activity is coincident with cell activation culminating in an inflammatory response. It is well known that cytosolic PLA₂ occupies a key receptor-mediated regulatory position in the biosynthetic pathways of potent lipid mediators of inflammation, hypersensitivity, and tissue injury.

The vast majority of PLA₂s isolated and studied thus far have been extracellular forms. Inhibitors identified in extracellular PLA₂ based models have not proven to be effective inhibitors of inflammation in vivo. To develop PLA₂ inhibitors with anti-inflammatory properties, it now appears that the more relevant intracellular PLA₂ involved in the inflammatory pathway should be used as the model enzyme rather than a more easily isolated extracellular PLA₂ (Dennis, Bio/Technology, 5: 1294, (1987)).

The ability to modulate receptor mediated, cytosolic PLA₂ activity via enzymatic inhibitors is a desirable goal and may lead to new therapies for the treatment of asthma, ischemia, arthritis, septic shock, and inflammatory diseases of the skin. The inactivation or specific inhibition of cytosolic PLA₂ activity associated with particular disease states will be of great use to the medical community. To accomplish this goal, cytosolic PLA₂s involved with the pathogenesis of certain diseases must first be identified and isolated.

The invention provides a polypeptide comprising the amino acid sequences: Leu-Lys-Leu-Asn-Pro-Thr-Ile-Ile-Tyr-Phe-Val-Leu-Ala-Asn-Ile-Asn-Phe-Arg-Lys-Tyr-Lys-Ala-Pro-Gly-Val-Pro, and Leu-Lys-Leu-Pro-Phe-Pro-Tyr-Val-Glu-Leu-Phe-Ile-Glu-Lys-Ile-Pro-Arg having phospholipase A₂ activity and a molecular weight of approximately 100 kilodaltons, and which attains maximum enzymatic activity at Ca²⁺ concentrations below 1 µM Also included in the invention is a polypeptide comprising the amino acid sequence: Leu-Lys-Leu-Asn-Pro-Thr-Ile-Ile-Tyr-Phe-Val-Leu-Ala-Asn-Ile-Asn-Phe-Arg-Lys-Tyr-Lys-Ala-Pro-Gly-Val-Pro having PLA₂ activity and a molecular weight of approximately 100 kilodaltons,and which attains maximum enzymatic activity at Ca²⁺ concentrations below 1 µM.

The invention further encompasses a polypeptide comprising the amino acid sequence: Leu-Lys-Leu-Pro-Phe-Pro-Tyr-Val-Glu-Leu-Phe-Ile-Glu-Lys-Ile-Pro-Arg having PLA₂ activity and a molecular weight of approximately 100 kilodaltons, and which attains maximum enzymatic activity at Ca²⁺ concentrations below 1 µM.

The invention contains a method of isolating a phospholipase A₂ in substantially pure form which is a polypeptide as defined above, from a biological cell, comprising:
a) lysing said cell;
b) fractionating the cell lysate to obtain a proteinaceous solution substantially free from cellular debris, organelles and membranes;
c) contacting said proteinaceous solution with an anion exchange resin capable of binding the phospholipase A₂ contained in the proteinaceous solution;
d) eluting said phospholipase A₂ from said anion exchange resin;
e) contacting the phospholipase A₂ containing solution obtained in d) with a hydrophobic interaction resin capable of binding said phospholipase A₂;
f) eluting said phospholipase A₂ from said hydrophobic interaction resin;
g) separating the phospholipase A₂ containing solution from co-eluting contaminants by molecular sizing chromatography;
h) contacting the phospholipase A₂ containing solution obtained in g) with a hydrophobic interaction resin under conditions that allow only weak interaction between the PLA₂ and the resin.

Figure 1 represents the results of an SDS polyacrylamide gel electrophoresis. The individual lanes show the purifying effect of each step of the process of the invention.

Figure 2 demonstrates that the purified protein of the invention directly correlates with maximal PLA₂ activity.

Figure 3 is a concentration effect graph showing that the PLA₂ of the invention is dependent on Ca²⁺ and attains maximum enzymatic activity at Ca²⁺ concentrations below 1 µM.

Figure 4 represents the cyanogen bromide cleavage products of the PLA₂ of the invention after being subjected to SDS polyacylamide gel electrophoresis.

In one embodiment, the present invention provides a process for a more than 10,000 fold purification of cytosolic PLA₂ from the human monoblast cell line U937. This novel form of PLA₂ is useful in models which identify compounds that possess anti-inflammatory and anti-ischemic properties. The current state of the art indicates that compounds which selectively inhibit a specific PLA₂ occupying a regulatory position in the formation of lysophospholipids and the biosynthetic pathway of eicosanoids and PAFs will be excellent candidates for anti-inflammatory and anti-ischemic drugs.

The process of the invention begins by lysing cells which contain cytosolic PLA₂. All cells contain some form of PLA₂, and the choice of which cell type to use depends largely on the cell's overall function in the body. With respect to cytosolic PLA₂s which participate in the regulation of eicosanoid and PAF production, cells that compose the immune system are preferred. More preferred cell types are those of the hematopoietic system. More highly preferred cells are those of myeloid origin which are directly involved in the inflammatory response. Platelets, neutrophils, macrophages, monocytes, and cell lines derived from such cells are examples of highly preferred cells. Cell lines are often not derived from the mature cell type; rather they are more often developed from cells which arise from the differentiating stem cell. The differentiation process includes all variety of cells that rise from the stem cell and culminates in the mature cell type. For purposes of this document, cells within the differentiation process will be referred to as precursor cells.

The chosen cell type must be secured in sufficient quantity to enable the purification of the particular cytosolic PLA₂ contained within the cell type. The required cell mass will of course depend on the concentration of the sought after PLA₂ within the cell. Because cytosolic PLA₂s are found in such minute concentrations within cells, 10¹¹ to 10¹² total cells are typically required to begin the process of the invention. Thus, in vitro cell culture is the preferred method of acquiring the large number of cells required to practice the invention. Such techniques are well known in the art and may be exercised in a variety of modes. However, isolating platelets from blood donations is also a preferred method of procuring cells to practice the process of the invention.

Once grown and harvested, the cells of choice may be lysed by various methods including but not limited to enzymatic degradation, chemical lysis, osmotic shock, mechanical disruption, sonication, and pressure shock. Preferred lytic methods are mechanical ones and pressure shock is especially preferred. Nitrogen cavitation is the most preferred form of pressure shock for disrupting the cell. Lysis by nitrogen cavitation is accomplished by placing the cell suspension in a stirred cell disruption bomb and pressurizing it with nitrogen to approximately 600 pounds per square inch for twenty minutes. The pressure is then suddenly released causing the cells to burst.

The lytic procedure (step a) is best performed in the presence of one or more protease inhibitors in a physiological buffer. In so doing, the denaturation and degradation of exceedingly scarce cytosolic PLA₂s is minimized. There are many different commercially available protease inhibitors. Examples include but are not limited to antipain, alpha₁-antitrypsin, aprotinin, bestatin, chymostatin, cystatin, leupeptin, pepstatin A, phenylmethanesulfonyl fluoride (PMSF), and trypsin inhibitor (Boehringer Mannheim, Indianapolis, IN). The preferred protease inhibitor combination is PMSF (1 mM), leupeptin (100 µM), and pepstatin A (100 µM) in the presence of 2 mM ethylenediaminetetraacetic acid.

In step b) soluble and particulate fractions are prepared from the lysate to facilitate subsequent purification steps. The elimination of cellular debris and organelles from the soluble fraction results in a solution of cytosolic proteins including PLA₂ which may be further purified. The preferred fractionation technique is ultracentrifugation though other methods of separation such as sequential filtration are consistent with the invention.

Step c) continues the isolation by contacting the PLA₂ containing solution with an anion exchange resin. Separation techniques that exploit the ionic charges of a molecule are well know in the art. A basic text describing the state of the art in protein purification which is useful for understanding the invention is Scopes, R.K., Protein Purification, Springer-Verlag New York Inc., 1982. Step b) may be performed in a batch mode; however, those skilled in the art will readily understand that this step is best accomplished by column chromatography (CC). Numerous anion exchange resins are commercially available and are compatible with the invention. Examples of such resins are Fast Flow Sepharose Q (Pharmacia), Cellfine A-800 (Amicon), and Silica PAE (Amicon). The most preferred resin is Fast Flow Sepharose Q. The preferred binding and washing buffer is 150 mM NaCl, 2 mM 2-mercaptoethanol (2-ME), 1 mM ethylene-bis(oxy-ethylenenitrilo)tetraacetic acid (EGTA), 25 mM Tris/HCl at pH 8.

Once the sought after PLA₂ has been ionically bound to the resin and contaminating proteins have been washed away, the PLA₂ must be eluted from the resin. Step d) is achieved by altering the ionic characteristics of the buffer so that buffer ions displace the PLA₂ from the resin. Changing the buffer to elution conditions in a stepwise manner is consistent with the process of the invention. However, those skilled in the art will immediately recognize that an elution buffer gradient is preferred because greater resolution between eluted proteins is achieved. The most preferred gradient begins with 100% binding and washing buffer and linearly advances towards 100% elution buffer (500 mM NaCl, 2 mM 2-ME, 1 mM EGTA, 25 mM Tris/HCl at pH 8). The column eluate is preferably monitored for absorbance at 280 nm as fractions are collected.

Fractions obtained from step d) should be assayed for PLA₂ activity and may be concentrated by standard techniques. Preferred concentration techniques will be gentle so as not to denature the enzyme and will preferably not concentrate solute ions. Standard concentration procedures employing molecular sizing and desalting gels such as Sephadex G-25 or G-50 as well as Biogel P30 or P60 are preferred. More highly preferred concentration methods are based on ultrafiltration techniques. Numerous ultrafiltration concentration units are commercially available such as a stirred cell with a YM 30 membrane (Amicon).

The concentrated sample containing PLA₂ is further purified in step e) by means of a hydrophobic interaction resin. Such resins are well known in the art and are used to purify proteins based on the particular hydrophobic properties inherent in the molecule of interest. Examples of such resins include but are not limited to Phenyl-Superose (Pharmacia), Phenyl-Sepharose (Pharmacia), and Hydropore HIC (Rainin). Under appropriate buffer conditions, PLA₂ will bind the hydrophobic interaction resin and, after washing away proteins that do not bind, can be eluted from the column without deleterious effects.

Those skilled in the art will understand that step e) is preferably accomplished by CC methods. The preferred hydrophobic interaction resin is Phenyl-Superose, and the preferred binding buffer is 500 mM NaCl, 2 mM 2-mM, 1 mM EGTA, 25 mM Tris/HCl at pH 8. It is preferred that the PLA₂ be eluted in step f) using a linear gradient starting with 100% binding buffer and progressing to 100% elution buffer (50% ethylene glycol in water, 2 mM 2-ME, 1 mM EGTA, 25 mM Tris/HCl at pH 8). The column eluate is preferably monitored for absorbance at 280 nm as fractions are collected. The resultant fractions should be assayed for PLA₂ activity and concentrated as discussed above before proceeding with subsequent steps of the invention.

Step g) of the process further purifies the PLA₂ containing sample using molecular sizing techniques. It is preferable to continue with this step only if a minimum of 5 mg of protein has been isolated up through step f). If pooling becomes necessary prior to step g), the isolated material may be stored at a concentration greater than 2 mg/ml at 4°c for up to three weeks without any adverse effects. Purification of enzymatically active proteins based on molecular size may be accomplished by a variety of methods including but not limited to filtration, dialysis, density gradients and gel electrophoresis.

The preferred molecular sizing method is by gel filtration. Numerous commercial resins and columns are available for this purpose and are consistent with the invention. Such resins include but are not limited to Superose 12, Superose 6, Sephadex G-200 (Pharmacia), and Bio-gel P-300 (BioRad). The most preferred resin is Superose 12. Many different buffers are acceptable for gel filtration methods depending on the characteristics of the molecule to be purified. The preferred gel filtration buffer for PLA₂ of the invention is 30% ethylene glycol in water, 150 mM NaCl, 2 mM 2-ME, 1 mM EGTA, 25 mM Tris/HCl at pH 8.

Prior to molecular sizing, the concentrated PLA₂ containing sample obtained in step g) is preferably solvent exchanged into the same buffer with which the molecular sizing column is pre-equilibrated. The PLA₂ containing sample can be solvent exchanged by a variety of well known techniques including but not limited to dialysis and dilution followed by concentration.

Step h) is the final step in the process and purifies the PLA₂ of the invention to homogeneity. The PLA₂ containing solution obtained in step g) is contacted with a hydrophobic interaction resin under buffer conditions that result in only weak interactions between the PLA₂ and the resin. The hydrophobic interaction resins and columns that were appropriate and preferred in step e) are consistent with this step. Using optimal buffer conditions, the progress of the PLA₂ through the resin is retarded while contaminating molecules either bind the resin or flow through the resin unimpeded. CC is the preferred method of accomplishing step h) as those skilled in the art will readily recognize. The preferred buffer in which the PLA₂ containing sample is prepared for step h) and with which the column is run is 150 mM NaCl, 2 mM 2-Me, 1 mM EGTA, 25 mM Tris/HCl, pH 8. It is important to note that the PLA₂ weakly binds the resin under these buffer conditions and exits the column during the wash phase. A gradient elution step following the washing procedure is not an essential step of the process but is necessary only to purge the column in preparation for its next use. As in the previous steps, the column fractions should be assayed for PLA₂ activity, and the fractions coinciding with peak activity are pooled and concentrated as discussed above.

Active cytosolic PLA₂ which has been purified to homogeneity by the process of the invention may be stored in a variety of ways. Preferred storage methods will not detrimentally affect the enzymatic activity of the purified PLA₂. Numerous storage methods are well known in the art and include storage in a preservative buffer or as a lyophilized powder at 4°C, -20°C or -70°C. The most preferred way to store the purified PLA₂ of the invention is at -20°C in a solution prepared by mixing one part of the final column buffer, which contains the PLA₂, with two parts of 75% glycerol / 25% 150 mM NaCl, 1mM EGTA, 25 mM Tris/HCl, pH 8. 2-ME was added to a final concentration of 2 mM.

The following examples will help describe how the process of the invention is practiced and will illustrate the characteristics of the invention's claimed PLA₂ enzymes.

### Example 1

### Culture of U937 Cells

The human leukemia monoblastoid cell line U937 was obtained from American Type Culture Collection (ATCC). This cell line is a permanent part of the collection and is available under accession number CRL 1593. U937 cells were maintained in culture in D-MEM/F-12 medium (custom, GIBCO Laboratories, Grand Island, New York) supplemented with 5% fetal bovine serum (HyClone Laboratories, Logan, Utah), 4 mM L-glutamine (Tanabe USA, San Diego, CA), 0.02% Pluronic F-68 (BASF Corporation, Parsippany, N.J.), 1 mM CaCl₂, 50 ug/ml Tobramycine (Eli Lilly and Company, Indianapolis, IN), 20 mM Hepes (USB, Cleveland, Ohio), 2.2 g/l NaHCO₃, 100 µM ethanolamine (Sigma Chemical, St. Louis, MO).

### A) Thawing Procedure:

The ampule from ATCC containing 1 ml of the cell suspension was removed from its dry ice container and placed into a 37°C water bath with vigorous agitation until the ice had melted. All operations from this point were carried out using sterile techniques. The ampule was opened, and the contents were removed and placed into a sterile 15 ml conical centrifuge tube. After adding 9 ml of medium (at 37°C), the tube was centrifuged trifuged at 1000 rpm for 10 minutes in a table top (Damon/IEC Division, Needham Heights, Mass.). The medium was decanted and the cell pellet resuspended in 50 ml of medium (37°C) and transferred to a T-75 tissue culture flask (Corning Glass, Corning, New York) that was placed in a 37°C incubator equilibrated to 5% CO₂, 95% air (Forma Scientific, Marietta, Ohio).

### B) Growth of Cells:

When the cell count reached 1-1.5 x 10⁶ cells/ml as determined by a Coulter Counter (Coulter Electronics, Hialeah, Florida), the culture was transferred into 450 ml fresh growth medium (500 ml total) in a 500 ml sterile microcarrier spinner flask (Bellco Biotechnology, Vineland, New Jersey) and stirred at 50 rpm using a microcarrier magnetic stirrer (Bellco) in a 5% CO₂ incubator at 37°C. When cell density reached 1-1.5 x 10⁶, the 500 ml cell suspension was placed into 5.5 liters of medium at 37°C in a 6 liter microcarrier spinner (Bellco). The spinner flask was fitted with 0.2 micron vent filters (Gelman Sciences, Ann Arbor, Michigan) so that the headspace could be gassed continuously with 5% carbon dioxide, 25% oxygen, and 70% nitrogen. Stirring was at 50 rpm at 37°C. When cell density reached 1-1.5 x 10⁶ cells/ml, the 5 liter cell suspension was placed into approximately 36 liters of medium at 37°C in a sterile 36 liter Biotech Overhead Drive System (Bellco). The culture was sparged with 70% nitrogen, 25% oxygen, and 5% carbon dioxide through a sterile vent filter. A Pasteur pipet was fitted through one of the side ports, and gas flow was set at 5 to 10 bubbles/second. The culture was stirred at 6-7 rpm and incubated at 37°C. Daily cell counts were performed and when cell density reached approximately 1 x 10⁶ cells/ml, the culture was sparged at a very slow rate with 100% oxygen.

### C) Harvest Procedure:

When the culture reached 2-3 x 10⁶ cells/ml, the contents of the growth vessel were transferred to a sterile 50 liter carboy (Nalgene, Rochester, New York). The carboy was capped and stored at 4°C for approximately 48 hours to allow the cells to settle to the bottom of the container. All subsequent steps were performed at 4°C and did not require sterile procedures. Most of the medium was aspirated from the top of the carboy and discarded, leaving 5-6 liters of cell suspension. The carboy was swirled to resuspend the cells in the medium, and the cell suspension was poured into six, 1 liter centrifuge bottles (Nalgene, Rochester, New York). The bottles were centrifuged for 30 minutes at 2500 rpm in an IEC centrifuge (Damon/IEC, Needham Heights, Mass.). The supernatant was discarded, and the cells were washed in 2 liters of 140 mM NaCl, 5 mM KCl, 2 mM ethylenediamine tetraacetic acid (EDTA), 25 mM Tris/HCl at pH 7.4. The washed cell pellets were combined and resuspended at 10⁸ cells/ml (approximately 10 mg/ml protein) in 150 mM NaCl, 2 mM EDTA, 50 mM Tris/HCl, 100 µM leupeptin at pH 8 yielding approximately 500 ml of cell suspension or 5 gm of cellular protein.

### Example 2

### Purification of Phospholipase A₂

### A) Preparation of U937 Cytosolic Fraction:

After addition of PMSF and pepstatin A to 1 mM and 100 µM (from a combined of stock in methanol of 200 mM and 20 mM, respectively), the cells were transferred to a precooled Parr cell disruption bomb (Parr Instr. Co., Moline, IL), mixed with a magnetic stirrer at 4°C for 20 minutes under 600 psi nitrogen and lysed by releasing the pressure. The cell lysate was centrifuged (150,000 x g, 60 min, 4°C) and the resulting supernatant collected. Prior to column chromatography this cell extract was filtered first through cheese cloth and then through a Millipak-40 filter (0.22 µm, Millipore, Bedford, MA) using a variable speed peristaltic pump.

### B) Sepharose Q Anion Exchange Chromatography:

The cytosolic fraction (2-3 gm protein) was applied to a 3.2 x 14 cm Fast Flow Sepharose Q (Pharmacia) column that had been pre-equilibrated with 150 mM NaCl, 2 mM 2-ME, 1 mM EGTA, 25 mM Tris/HCl, pH 8 at a flow rate of 6 ml/min. The column was washed with 500 ml of the same buffer, and elution proceeded by developing a 1500 ml linear salt gradient from 150 mM to 500 mM NaCl in 2 mM 2-ME, 1 mM EGTA, 25 mM Tris/HCl, pH 8 collecting fractions of 12 ml. The column eluate was monitored for absorbance at 280 nm (AUF 2.0) and 2 ul aliquots of each fraction were assayed for PLA₂ activity. The PLA₂ eluted with approximately 380 mM NaCl at fractions 47-56 (120 ml). These fractions were pooled and concentrated to 25 ml using an Amicon ultrafiltration stirred cell with a YM 30 membrane.

### C) Hydrophobic Interaction Chromatography:

The concentrated peak fractions (150 mg of protein) from the anion exchange column were applied to a Phenyl-Superose HR 10/10 column (Pharmacia) that had been pre-equilibrated with 500 mM NaCl, 2 mM 2-ME, 1 mM EGTA, 25 mM Tris/HCl, pH 8 at a flow rate of 1 ml/min collecting 4 ml fractions. After washing the column with 120 ml of the same buffer, a 120 ml linear gradient was applied at a flow rate of 0.75 ml/min using one part of column buffer and one part of elution buffer (50% ethylene glycol, 2 mM 2-ME, 1 mM EGTA, 25 mM Tris/HCl, pH 8). The column eluate was monitored for absorbance at 280 nm and 2 µl aliquots of each fraction was assayed for PLA₂ activity. The PLA₂ eluted as a broad peak from 15-45% ethylene glycol in fractions 32-52 (40 ml). These fractions were pooled, concentrated to first an Amicon stirred cell with a YM 30 membrane and then Centricon-30 microconcentrators (Amicon), resuspended in 15 volumes of 30% ethylene glycol, 2 mM 2-ME, 150 mM NaCl, 1 mM EGTA, 25 mM Tris/HCl, pH 8 and re-concentrated to 2 ml.

### D) Superose 12 Gel Filtration:

The concentrated peak fractions from the hydrophobic interaction column were pooled (approximately 5 mg protein) and chromatographed on tandem Superose 12 columns (each 1.6 x 50 cm) pre-equilibrated with 30% ethylene glycol, 2 mM 2-ME, 150 mM NaCl, 1 mM EGTA, 25 mM Tris/HCl, pH 8 at a flow rate of 0.15 ml/min collecting 1.5 ml fractions. The column eluate was monitored for absorbance at 280 nm, and 2 µl aliquots of each fraction were assayed for PLA₂ activity. The enzymatic activity eluted in fractions 38-47 (15 ml). These fractions were pooled and concentrated to microconcentrators (Amicon). The concentrated samples (approximately 80 µl per concentrator) were each resuspended in 2.5 ml of 150 mM NaCl, 2 mM 2-ME, 1 mM EGTA, 25 mM Tris/HCl, pH 8 (to give a final ethylene glycol concentration of < 1%) and reconcentrated to approximately 80 µl then pooled resulting in a final volume of < 650 µl.

### E) Hydrophobic Interaction Chromatography:

The concentrated sample (approximately 1 mg protein) from the gel filtration column was diluted to 1 ml using 150 mM NaCl, 1 mM 2-ME, 1 mM EGTA, 25 mM Tris/HCl, pH 8. The sample was loaded onto a Phenyl-Superose HR 5/5 (Pharmacia) column that had been preequilibrated with the same buffer used for the dilution at a flow rate of 0.25 ml/min, and 0.5 ml fractions were collected. After washing the column with 8 ml of the same buffer a 10 ml linear gradient was applied at a flow rate of 0.25 ml/min using one part of column buffer and one part of elution buffer (50% ethylene glycol, 2 mM 2-ME, 1 mM EGTA, 25 mM Tris/HCl, pH 8). As discussed previously, elution is only necessary to recondition the column for subsequent use. The column eluate was monitored for absorbance at 280 nm, and a 2 µl aliquot of each fraction was assayed for PLA₂ activity. The PLA₂ activity appeared in fractions 7-20 (7 ml). These fractions were pooled, concentrated to approximately 60 µl using first Centricon-30 microconcentrators (Amicon), then an Ultrafree-MC 10,000 NMWL filter unit (Millipore, Bedford, MA). The recovery of protein was approximately 100 µg.

### F) Results:

Table 1 summarizes the results of the process of the invention as illustrated by Examples 1 and 2. The data was generated from combined purifications taking two, 40 liter preparations through step f) then combining them for steps g) and h).

**TABLE 1**

| Claim 1 Steps | | Protein (mg tot) | Specific PLA₂ (mU/mg) | Total PLA₂ (mU) | Yield (%) | Purific. (-fold) |
|---|---|---|---|---|---|---|
| a) | N₂ cavitation | 15621 | 0.05 | 748 | - | - |
| b) | UC 150,000xg | 6066 | 0.31 | 1882 | 100 | 3 |
| c) & d) | Q-Sepharose | 293 | 3.74 | 1095 | 58 | 31 |
| e) & f) | Phenyl-Superose | 6.3 | 76.0 | 529 | 29 | 630 |
| g) | Superose 12 | 0.5 | 517 | 265 | 14 | 4289 |
| h) | Phenyl-Superose | <0.1 | >1722 | 172 | 9 | >14278 |

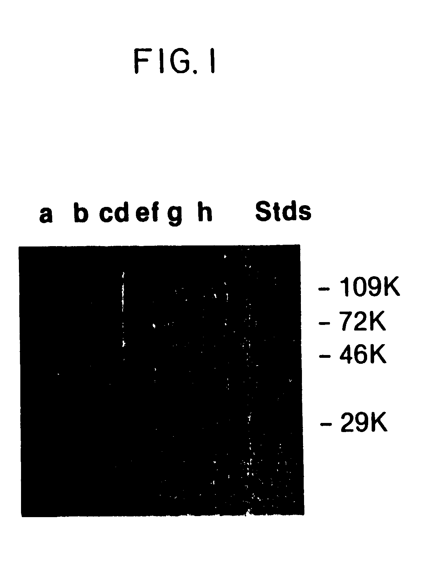

Figure 1 is a reproduction of a 12% SDS polyacrylamide gel electrophoresis (SDS-PAGE) run under reducing conditions according to Laemmli (Laemmli, Nature, 227: 680 (1970)). Each lane of the gel shows a pattern of proteins that have been isolated as a result of each purification step of the invention. Lane 7 demonstrates that the PLA₂ isolated as a result of the process of the invention is substantially pure and has a molecular weight of approximately 100 Kd.

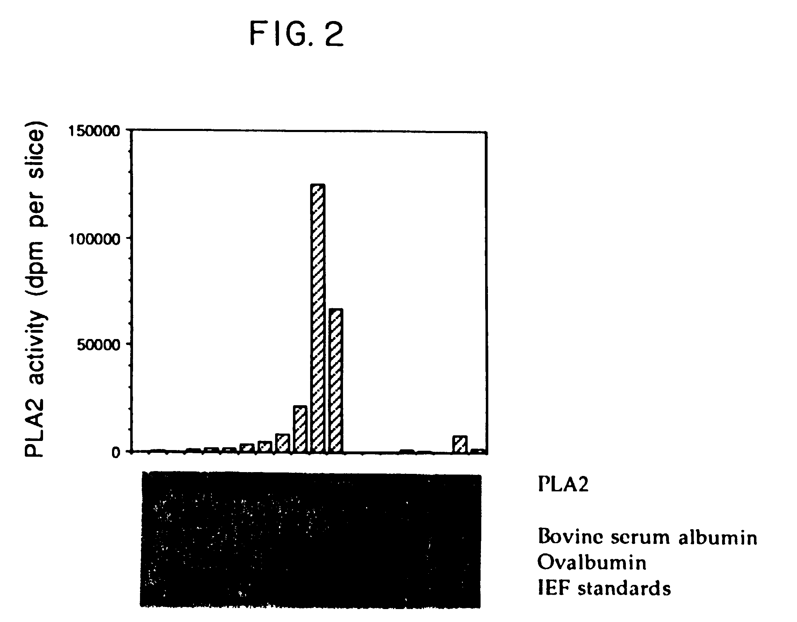

Figure 2 represents a non-denaturing polyacrylamide gel electrophoresis of the purified PLA₂ of the invention. A parallel unstained PLA₂ lane was cut into 20 slices and the contents of each slice was eluted from the gel using 1% CHAPS, 150 mM NaCl, 2 mM 2-ME, 1 mM EGTA, 25 mM Tris/HCl, pH 8 plus 2 mg/ml bovine serum albumin at 4°C over night. The eluates were placed in a PLA₂ activity assay to demonstrate that the protein purified by the process of the invention is in fact a PLA₂ enzyme. Figure 2 shows that the primary protein band on the gel clearly coincided with peak PLA₂ activity.

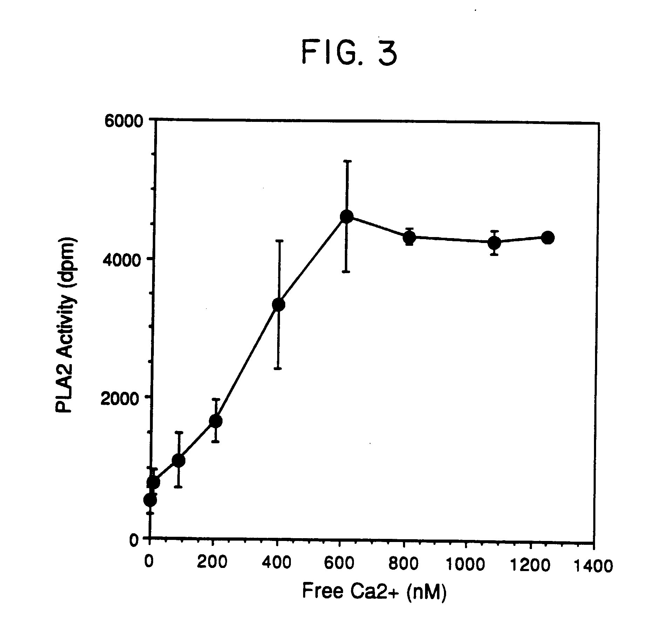

Figure 3 depicts the results of a PLA₂ activity assay where the Ca²⁺ requirements of the isolated product were examined. The plotted curve represents two different assays which clearly show that the PLA₂ of the invention requires Ca²⁺ for activity and reaches maximum enzymatic activity at approximately 800 nM Ca²⁺ concentration. This finding is unique to the PLA₂ of the invention because all other well characterized low molecular weight PLA₂s reported to date have maximum activity above 1 mM Ca²⁺ concentration.

### EXAMPLE 3

### Amino Acid Sequencing

A pilot experiment established that the amino terminus of the isolated PLA₂ was blocked thus not permiting N-terminal amino acid sequencing. This finding was not unexpected because it is well known that cytoplasmic proteins are often blocked from this type of analysis. Partial amino acid sequences were obtained by cleaving the enzyme with cyanogen bromide (CNBr) after SDS-PAGE. The cleaved PLA₂ was then rerun on SDS-PAGE, electroblotted to a protein binding membrane, and then subjected to amino acid sequence analysis.

### A) SDS-PAGE:

After mixing 20 µl of concentrated (4x) sample buffer (8% SDS, 40% glycerol, 20% 2-ME and 400 mM Tris/HCl, pH 6.8) with 60 µl of the concentrated fractions from the Phenyl-Superose 5/5 column and incubating at 60°C for 20 minutes, the samples were subjected to SDS-PAGE according to Laemmli. Aliquots of 20 µl (approximately 25 µg protein) were loaded onto a minigel (5 x 7 cm) containing a 9% SDS polyacrylamide separating gel and a 5% stacking gel and electrophoresed at 20 mA constant current for 90 minutes. Following electrophoresis, the PLA₂ band was visualized using a one-step, low background Coomassie staining procedure (B.D. Zehr, T.J. Savin and R.E. Hall, Anal. Biochem., 182: 157, (1989)) and excised from the gel. Each gel slice was washed twice with 1 ml of 10% acetic acid, 5% methanol and stored in Eppendorf tubes at -20°C.

### B) Cyanogen Bromide Cleavage:

Each polyacrylamide gel slice containing PLA₂ of the invention was washed for 10 minutes with 1 ml of water and then exposed to CNBr (100 mg/ml) in 1 ml of 0.1 N HCl containing 20% formic acid. The CNBr was diluted from a freshly prepared stock of 500 mg/ml in 90% formic acid. Unreacted CNBr was removed by extensive sequential washing. One 5 minute incubation with 1 ml of water, two 5 minute incubations with 1 ml of 100 mM Tris/HCl, pH 8.8, one 5 minute incubation with 1 ml of 100 mM Tris/HCl, pH 6.8 and one 10 minute incubation at 37°C with 400 µl of sample buffer (2% SDS, 10% glycerol, 5% 2-ME and 100 mM Tris/HCl, pH 6.8 containing 0.002% bromphenol blue) removed the CNBr from the gel slices. The slices were stored at -70°C until they could be electrophoresed.

### C) SDS Polyacrylamide Gel Electrophoresis and Protein Transfer:

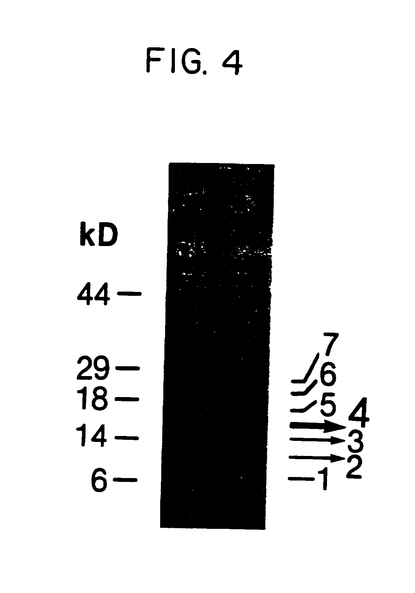

Proteins from the gel slices were electrophoresed into SDS polyacrylamide gels containing a 5% stacking gel and a 14% separating gel. Following electrophoresis, the gel was soaked in transfer buffer (10 mM 3-[cyclohexylamino]-1-propanesulfonic acid, 10% methanol, pH 11) for 5 minutes and electroblotted at 4°C onto a polyvinylidene difluoride (PVDF) membrane (Immobilon; 45 um pore size, Millipore, Bedford MA) for 2 hours at 250 mA (P. Matsudaira, J. Biol.Chem., 262: 10035 (1987)). The CNBr peptides were visualized by staining the PVDF membrane with Coomassie Blue R-250. The membrane was rinsed extensively with deionized water, dried and stored at -20°C. Figure 4 is a representation of the transferred cleavage products and molecular weight standards. Seven distinct bands are visible in the PLA₂ lane indicating that the PLA₂ of the invention was only partially cleaved by the CNBr treatment.

### C) Amino Acid Sequence Analysis:

Three major bands (peptides 2, 3 and 4) visualized on the PVDF membrane following electrophoresis and electroblotting were excised and subjected to automated Edman degradation using an Applied Biosystems 477A Pulsed Liquid Sequencer equipped with a model 900A data system (R. M. Hewick et al., J.Biol.Chem., 256: 7990 (1981)). The PVDF membranes containing the blotted CNBr peptide (derived from the entire PLA₂ preparation) were placed on top of a polybrene-conditioned filter as suggested by the manufacturer of the protein sequencer. The resulting phenylthiohydantoin amino acids were analyzed on-line using an Applied Biosystems 120A PTH amino acid analyzer equipped with a PTH-C18 column (2.1 x 220 mm). The 26 amino-terminal amino acids for peptide 4 were Leu-Lys-Leu-Asn-Pro-Thr-Ile-Ile-Tyr-Phe-Val-Leu-Ala-Asn-Ile-Asn-Phe-Arg-Lys-Tyr-Lys-Ala-Pro-Gly-Val-Pro. A tentative sequence was assigned to peptide 3: Leu-Lys-Leu-Pro-Phe-Pro-Tyr-Val-Glu-Leu-Phe-Ile-Glu-Lys-Ile-Pro-Arg. Peptide 2 appeared to give the same sequence as peptide 4.

### EXAMPLE 4

### PLA₂ Activity Assay

The substrate, sonicated liposomes containing 1-palmitoyl-2-[14C]arachidonoyl-sn-glycero-3-phosphocholine ([14C]PC, 55 mCi/mmol from NEN Research Products) and sn-1,2-dioleoylglycerol (DG, Avanti Polar Lipids, Birmingham, AL) at a molar ratio of 2:1, was prepared as follows. [14C]PC (20 nmol, 1 x 10⁶ dpm, 100 µCi/ml in toluene/ethanol) and DG (10 nmol, 100 µg/ml in chloroform) were dried under nitrogen. The lipids were dispersed in 1 ml of 150 mM NaCl, 50 mM Hepes, pH 7.5 (assay buffer) by sonication at 4°C with a Microson probe-sonicator (Heat-systems, Ultrasonics) for 4 x 15 second, with 45 second intervals. Bovine serum albumin (essentially fatty acid free, from a 100 mg/ml stock in water, Sigma) was added to a final concentration of 4 mg/ml. Samples to be assayed for PLA₂ activity were incubated with 50 µl liposomes (0.5 nmol [14C]PC, 50,000 cpm containing 0.25 nmol of DG) in a total volume of 0.2 ml of assay buffer containing 1 mM CaCl₂ and 1 mM 2-ME. Incubations were carried out at 37°C for 15 minutes and terminated by adding 2 ml of Dole's reagent ((2-propanol/heptane/0.5 M sulfuric acid, 40:10:1 containing 10 ug/ml of stearic acid). After mixing 1.2 ml of heptane and 1 ml of water were added. The mixtures were briefly vortexed and the upper phase transferred to tubes containing 2 ml of heptane and 150 mg of Bio-Sil (Bio-Rad Laboratories) activated at 130°C before use. The tubes were thoroughly vortexed and centrifuged (1000 x g for 5 minutes). The supernantants were decanted into scintillation vials.

After addition of 10 ml of a liquid scintillation cocktail (Ready Protein⁺, Beckman) radioactivity was counted using a Packard liquid scintillation counter Model 1500. High radioactive counts correlate with enzymatic activity.

## Claims (Claims for the following Contracting State(s): AT, BE, CH, DE, DK, FR, GB, GR, IT, LI, NL, SE)

1. A polypeptide comprising the amino acid
sequence: Leu-Lys-Leu-Asn-Pro-Thr-Ile-Ile-Tyr-Phe-Val-Leu-Ala-Asn-Ile-Asn-Phe-Arg-Lys-Try-Lys-Ala-Pro-Gly-Val-Pro having phospholipase A₂ activity and molecular weight of approximately 100 kilodaltons, and which attains maximum enzymatic activity at Ca²⁺ concentrations below 1 µM.

2. A polypeptide comprising the amino acid
sequence: Leu-Lys-Leu-Pro-Phe-Pro-Tyr-Val-Glu-Leu-Phe-Ile-Glu-Lys-Ile-Pro-Arg having phospholipase A₂ activity and molecular weight of approximately 100 kilodaltons, and which attains maximum enzymatic activity at Ca²⁺ concentrations below 1 µM.

3. A polypetide according to either of claims 1 and 2 comprising the amino acid
sequences: Leu-Lys-Leu-Asn-Pro-Thr-Ile-Ile-Tyr-Phe-Val-Leu-Ala-Asn-Ile-Asn-Phe-Arg-Lys-Try-Lys-Ala-Pro-Gly-Val-Pro and Leu-Lys-Leu-Pro-Phe-Pro-Tyr-Val-Glu-Leu-Phe-Ile-Glu-Lys-Ile-Pro-Arg having PLA₂ activity, a molecular weight of approximately 100 kilodaltons, and which attains maximum enzymatic activity at Ca²⁺ concentrations below 1 µM.

4. A method for isolating in substantially pure form cytosolic phospholipase A₂ which is a polypeptide according to any of claims 1 to 3, from a biological cell, comprising:
a) lysing said cell;
b) fractionating the cell lysate to obtain a proteinaceous solution substantially free from cellular debris, organelles and membranes;
c) contacting said proteinaceous solution with an anion exchange resin capable of binding the phospholipase A₂ contained in the proteinaceous solution;
d) eluting said phospholipase A₂ from said anion exchange resin;
e) contacting the phospholipase A₂ containing solution obtained in d) with a hydro-phobic interaction resin capable of binding said phospholipase A₂;
f) eluting said phospholipase A₂ from said hydrophobic interaction resin;
g) separating the phospholipase A₂ containing solution from co-eluting contaminants by molecular sizing chromatography;
h) contacting the phospholipase A₂ containing solution obtained in g) with a hydro-phobic interaction resin under conditions that allow only weak interaction between the PLA₂ and the resin.

5. The method of Claim 4 wherein said biological cell is a U937 human leukemia monoblastoid cell.

## Claims (Claims for the following Contracting State(s): ES)

1. A method for isolating in substantially pure form cytosolic phospholipase A₂, which is a polypeptide comprising the amino acid sequence:
Leu-Lys-Leu-Asn-Pro-Thr-Ile-Ile-Tyr-Phe-Val-Leu-Ala-Asn-Ile-Asn-Phe-Arg-Lys-Try-Lys-Ala-Pro-Gly-Val-Pro or Leu-Lys-Leu-Pro-Phe-Pro-Tyr-Val-Glu-Leu-Phe-Ile-Glu-Lys-Ile-Pro-Arg having PLA₂ activity, a molecular weight of approximately 100 kilodaltons, and which attains maximum enzymatic activity at Ca²⁺ concentrations below 1 µM, from a biological cell, comprising:
a) lysing said cell;
b) fractionating the cell lysate to obtain a proteinaceous solution substantially free from cellular debris, organelles and membranes;
c) contacting said proteinaceous solution with an anion exchange resin capable of binding the phospholipase A₂ contained in the proteinaceous solution;
d) eluting said phospholipase A₂ from said anion exchange resin;
e) contacting the phospholipase A₂ containing solution obtained in d) with a hydro-phobic interaction resin capable of binding said phospholipase A₂;
f) eluting said phospholipase A₂ from said hydrophobic interaction resin;
g) separating the phospholipase A₂ containing solution from co-eluting contaminants by molecular sizing chromatography;
h) contacting the phospholipase A₂ containing solution obtained in g) with a hydro-phobic interaction resin under conditions that allow only weak interaction between the PLA₂ and the resin.

2. The method of Claim 1 wherein said biological cell is a U937 human leukemia monoblastoid cell.

## Patentansprüche (Patentansprüche für folgende(n) Vertragsstaat(en): AT, BE, CH, DE, DK, FR, GB, GR, IT, LI, NL, SE)

1. Polypeptid, das die folgende Aminosäuresequenz umfaßt: Leu-Lys-Leu-Asn-Pro-Thr-Ile-Ile-Tyr-Phe-Val-Leu-Ala-Asn-Ile-Asn-Phe-Arg-Lys-Tyr-Lys-Ala-Pro-Gly-Val-Pro, Phospholipase A₂ Aktivität und ein Molekulargewicht von etwa 100 Kilodalton aufweist, und das eine maximale Enzymaktivität bei Ca²⁺ Konzentrationen unter 1 µM erreicht.

2. Polypeptid, das die folgende Aminosäuresequenz umfaßt; Leu-Lys-Leu-Pro-Phe-Pro-Tyr-Val-Glu-Leu-Phe-Ile-Glu-Lys-Ile-Pro-Arg, das Phospholipase A₂ Aktivität und ein Molekulargewicht von etwa 100 Kilodalton aufweist, und das eine maximale Enzymaktivität bei Ca²⁺ Konzentrationen unter 1 µM erreicht.

3. Polypeptid nach einem der Ansprüche 1 und 2, das die folgenden Aminosäuresequenzen umfaßt: Leu-Lys-Leu-Asn-Pro-Thr-Ile-Ile-Tyr-Phe-Val-Leu-Ala-Asn,Ile-Asn-Phe-Arg-Lys-Tyr-Lys-Ala-Pro-Gly-Val-Pro und Leu-Lys-Leu-Pro-Phe-Pro-Tyr-Val-Glu-Leu-Phe-Ile-Glu-Lys-Ile-Pro-Arg, eine PLA₂ Aktivität und ein Molekulargewicht von etwa 100 Kilodalton aufweist und das eine maximale Enzymaktivität bei Ca²⁺ Konzentrationen unter 1 µM erreicht.

4. Verfahren zur Isolierung von cytosolischer Phospholipase A₂, die ein Polypeptid nach einem der Ansprüche 1 bis 3 ist, aus einer biologischen Zelle in im wesentlichen reiner Form, gekennzeichnet durch
a) Lysieren dieser Zelle,
b) Fraktionierung des Zellysats, um eine proteinhaltige Lösung zu erhalten, die im wesentlichen frei ist von Zelldebris, Organellen und Membranen,
c) Zusammenbringen dieser proteinhaltigen Lösung mit einem Anionenaustauscherharz, das zur Bindung der in der proteinhaltigen Lösung enthaltenen Phospholipase A₂ fähig ist,
d) Elution dieser Phospholipase A₂ von diesem Anionenaustauscherharz,
e) Zusammenbringen der die Phospholipase A₂ enthaltenden Löung, die in d) erhalten wurde, mit einem hydrophoben Wechselwirkungsharz, das zur Bindung dieser Phospholipase A₂ fähig ist,
f) Elution dieser Phospholipase A₂ von diesem hydrophoben Wechselwirkungsharz,
g) Abtrennung der die Phospholipase A₂ enthaltenden Lösung von miteluierenden Kontaminationen durch Molekulargrößenchromatographie,
h) Zusammenbringen der die Phospholipase A₂ enthaltenden Lösung, die in g) erhalten wurde, mit einem hydrophoben Wechselwirkungsharz unter Bedingungen, die nur eine schwache Wechselwirkung zwischen der PLA₂ und dem Harz erlauben.

5. Verfahren nach Anspruch 4, worin diese biologische Zelle eine humane monoblastoide U937 Leukämiezelle ist.

## Patentansprüche (Patentansprüche für folgende(n) Vertragsstaat(en): ES)

1. Verfahren zur Isolierung von cytosolischer Phospholipase A2 in im wesentlichen reiner Form, die ein Polypeptid ist, das die folgende Aminosäuresequenz umfaßt: Leu-Lys-Leu-Asn-Pro-Thr-Ile-Ile-Tyr-Phe-Val-Leu-Ala-Asn-Ile-Asn-Phe-Arg-Lys-Tyr-Lys-Ala-Pro-Gly-Val-Pro oder Leu-Lys-Leu-Pro-Phe-Pro-Tyr-Val-Glu-Leu-Phe-Ile-Glu-Lys-Ile-Pro-Arg, eine PLA₂ Aktivität und ein Molekulargewicht von etwa 100 Kilodalton aufweist und das eine maximale Enzymaktivität bei Ca²⁺ Konzentrationen unter 1 µM erreicht, gekennzeichnet durch
a) Lysieren dieser Zelle,
b) Fraktionierung des Zellysats, um eine proteinhaltige Lösung zu erhalten die im wesentlichen frei ist von Zelldebris, Organellen und Membranen,
c) Zusammenbringen dieser proteinhaltigen Lösung mit einem Anionenaustauscherharz, das zur Bindung der in der proteinhaltigen Lösung enthaltenen Phospholipase A₂ fähig ist,
d) Elution dieser Phospholipase A₂ von diesem Anionenaustauscherharz,
e) Zusammenbringen der die Phospholipase A₂ enthaltenden Lösung, die in d) erhalten wurde, mit einem hydrophoben Wechselwirkungsharz, das zur Bindung dieser Phospholipase A₂ fähig ist,
f) Elution dieser Phospholipase A₂ von diesem hydrophoben Wechselwirkungsharz.
g) Abtrennung der die Phospholipase A₂ enthaltenden Lösung von miteluierenden Kontaminationen durch Molekulargrößenchromatographie,
h) Zusammenbringen der die Phospholipase A₂ enthaltenden Lösung, die in g) erhalten wurde, mit einem hydrophoben Wechselwirkungsharz unter Bedingungen, die nur eine schwache Wechselwirkung zwischen der PLA₂ und dem Harz erlauben.

2. Verfahren nach Anspruch 1, worin diese biologische Zelle eine humane monoblastoide U937 Leukämiezelle ist.

## Revendications (Revendications pour l'(les) Etat(s) contractant(s) suivant(s): AT, BE, CH, DE, DK, FR, GB, GR, IT, LI, NL, SE)

1. Polypeptide comprenant la séquence d'acides aminés : Leu-Lys-Leu-Asn-Pro-Thr-Ile-Ile-Tyr-Phe-Val-Leu-Ala-Asn-Ile-Asn-Phe-Arg-Lys-Tyr-Lys-Ala-Pro-Gly-Val-Pro présentant une activité de phospholipase A₂ et un poids moléculaire d'environ 100 kilodaltons, et qui atteint une activité enzymatique maximale à des concentrations en Ca²⁺ inférieures à 1 µM.

2. Polypeptide comprenant la séquence d'acides aminés : Leu-Lys-Leu-Pro-Phe-Pro-Tyr-Val-Glu-Leu-Phe-Ile-Glu-Lys-Ile-Pro-Arg présentant une activité de phospholipase A₂ et un poids moléculaire d'environ 100 kilodaltons, et qui atteint une activité enzymatique maximale à des concentrations en Ca²⁺ inférieures à 1 µM.

3. Polypeptide selon l'une ou l'autre des revendications 1 et 2, comprenant les séquences d'acides aminés : Leu-Lys-Leu-Asn-Pro-Thr-Ile-Ile-Tyr-Phe-Val-Leu-Ala-Asn-Ile-Asn-Phe-Arg-Lys-Tyr-Lys-Ala-Pro-Gly-Val-Pro et Leu-Lys-Leu-Pro-Phe-Pro-Tyr-Val-Glu-Leu-Phe-Ile-Glu-Lys-Ile-Pro-Arg présentant une activité de PLA₂, un poids moléculaire d'environ 100 kilodaltons, et qui atteint une activité enzymatique maximale à des concentrations en Ca²⁺ inférieures à 1 µM.

4. Méthode d'isolement d'une phospholipase A₂ cytosolique sous forme essentiellement pure, qui est un polypeptide selon l'une quelconque des revendications 1 à 3, à partir d'une cellule biologique, comprenant :
a) la lyse de ladite cellule;
b) le fractionnement du lysat cellulaire de façon à obtenir une solution protéinique essentiellement exempte de débris cellulaires, d'organelles et de membranes;
c) la mise en contact de ladite solution protéinique avec une résine d'échange anionique capable de lier la phospholipase A₂ contenue dans la solution protéinique;
d) l'élution de ladite phospholipase A₂ à partir de ladite résine d'échange anionique;
e) la mise en contact de la solution contenant la phospholipase A₂ obtenue à l'étape d) avec une résine d'interaction hydrophobe capable de lier ladite phospholipase A₂;
f) l'élution de ladite phospholipase A₂ de ladite résine d'interaction hydrophobe;
g) la séparation de la solution contenant la phospholipase A₂ des contaminants de co-élution par chromatographie d'exclusion de taille;
h) la mise en contact de la solution contenant la phospholipase A₂ obtenue à l'étape g) avec une résine d'interaction hydrophobe dans des conditions qui permettent uniquement une faible interaction entre la PLA₂ et la résine.

5. Méthode selon la revendication 4, dans laquelle ladite cellule biologique est une cellule monoblastoïde de leucémie humaine U937.

## Revendications (Revendications pour l'(les) Etat(s) contractant(s) suivant(s): ES)

1. Méthode d'isolement d'une phospholipase A₂ cytosolique sous forme essentiellement pure, qui est un polypeptide comprenant la séquence d'acides aminés : Leu-Lys-Leu-Asn-Pro-Thr-Ile-Ile-Tyr-Phe-Val-Leu-Ala-AsnIle-Asn-Phe-Arg-Lys-Tyr-Lys-Ala-Pro-Gly-Val-Pro ou Leu-Lys-Leu-Pro-Phe-Pro-Tyr-Val-Glu-Leu-Phe-Ile-Glu-Lys-Ile-Pro-Arg présentant une activité de PLA₂, un poids moléculaire d'environ 100 kilodaltons, et qui atteint une activité enzymatique maximale à des concentrations en Ca²⁺ inférieures à 1 µM, à partir d'une cellule biologique, comprenant:
a) la lyse de ladite cellule;
b) le fractionnement du lysat cellulaire de façon à obtenir une solution protéinique essentiellement exempte de débris cellulaires, d'organelles et de membranes;
c) la mise en contact de ladite solution protéinique avec une résine d'échange anionique capable de lier la phospholipase A₂ contenue dans la solution protéinique;
d) l'élution de ladite phospholipase A₂ à partir de ladite résine d'échange anionique;
e) la mise en contact de la solution contenant la phospholipase A₂ obtenue à l'étape d) avec une résine d'interaction hydrophobe capable de lier ladite phospholipase A₂;
f) l'élution de ladite phospholipase A₂ de ladite résine d'interaction hydrophobe;
g) la séparation de la solution contenant la phospholipase A₂ des contaminants de co-élution par chromatographie d'exclusion de taille;
h) la mise en contact de la solution contenant la phospholipase A₂ obtenue à l'étape g) avec une résine d'interaction hydrophobe dans des conditions qui permettent uniquement une faible interaction entre la PLA₂ et la résine.

2. Méthode selon la revendication 1, dans laquelle ladite cellule biologique est une cellule monoblastoïde de leucémie humaine U937.
